(19) Europäisches Patentamt
European Patent Office
Office européen des brevets

(11) **EP 3 566 720 A1**

(12) **EUROPEAN PATENT APPLICATION**

(43) Date of publication:
**13.11.2019 Bulletin 2019/46**

(51) Int Cl.:
**A61K 47/69** (2017.01)   **A61K 9/51** (2006.01)
**A61K 49/18** (2006.01)   **B82Y 5/00** (2011.01)

(21) Application number: **18171351.2**

(22) Date of filing: **08.05.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Max-Planck-Gesellschaft zur Förderung der Wissenschaften e.V.**
**80539 München (DE)**

(72) Inventors:
• **Landfester, Katharina**
  **55122 Mainz (DE)**
• **Mailänder, Volker**
  **55122 Mainz (DE)**

(74) Representative: **Henkel & Partner mbB**
**Patentanwaltskanzlei, Rechtsanwaltskanzlei**
**Maximiliansplatz 21**
**80333 München (DE)**

(54) **NANOPARTICLES WITH NON-COVALENTLY BOUND TARGETING MOIETIES FOR USE IN A THERAPEUTIC METHOD AND FOR NON-MEDICAL USE**

(57)    The present invention relates to a modified nanoparticle for use in a therapeutic method, wherein the therapeutic method comprises the administration of the modified nanoparticle to an organism, and wherein the modified nanoparticle is obtainable by a process comprising the steps of i) providing a nanoparticle and ii) contacting the nanoparticle with one or more targeting moieties at a pH value of less than 7.0 so as to non-covalently bind the one or more targeting moieties onto the surface of the nanoparticle, wherein the nanoparticle provided in step i) is made of a material having at least one protonable or deprotonable group on the surface thereof and/or the one or more targeting moieties contacted with the nanoparticle in step ii) has at least one protonable or deprotonable group.

**Description**

The present invention relates to a modified nanoparticle with targeting moieties, in particular cell-targeting moieties, on the surface thereof, to its use in a therapeutic method, such as for drug delivery, to its non-medical use and to a method for preparing such a modified nanoparticle.

Nanoparticles are used in various technical fields, such as in the electronical field, particularly in semiconductor technology, in chemistry and in medicine. Since they are small enough to circulate in blood vessels, nanoparticles are of particular interest for surface adhesion, cell-targeting and particularly for drug delivery, i.e. for delivering a drug to a specific site, organ or cell type of a patient. For this purpose, nanoparticles are filled with a drug or the drug is bound to the surface of the nanoparticles. In addition, the nanoparticles are modified by binding onto their surfaces one or more cell-targeting moieties, such as one or more antibodies, sugars or other ligands, which are able to recognize proteins on the surface of the target cells. For instance, if the drug shall be delivered to or into a specific cell (e.g. a dendritic cell), the cell-targeting moiety may suitably be an antibody having a strong affinity to at least one protein being exposed on the surface of the cell and being specific for this cell. When the so modified nanoparticles are injected into the blood of a patient, the modified nanoparticles shall be targeted by the targeting moieties to the cells and then optionally taken up into the cells. Thereby, the drug shall be delivered to the site, such as cell type, where it shall act.

Typically, the targeting moieties are coupled by covalent chemistry onto the surfaces of the nanoparticles. Different coupling chemistries are known to covalently bind proteins, such as antibodies, onto the surface of nanoparticles. The process of chemical coupling a targeting moiety onto the surface of a nanoparticle is, however, tedious and the targeting moiety, such as an antibody, sometimes loses its ability to bind to the antigen.

When modified nanoparticles are exposed to blood plasma or injected into the blood of an organism, proteins being present in the blood plasma or blood, respectively, absorb onto the surfaces of the modified nanoparticles, thus forming the so-called protein corona. These proteins often partially or even completely cover or mask, respectively, the chemically coupled targeting moieties of the nanoparticles, which significantly reduces or even completely avoids a cell-targeting of the modified nanoparticles as consequence thereof. It has been shown that the formation of the protein corona cannot even be completely suppressed if the targeting moieties are covalently bound to the surfaces of the nanoparticles by means of polyethylene glycol spacers or other spacers.

It has been already generally suggested to attach targeting moieties onto nanoparticles by means of non-covalent binding. However, the experiments made by the inventors of the present patent application have shown that such nanoparticles obtained by non-covalently binding targeting moieties to nanoparticles following usual protocols at physiological pH value have even a worse targeting efficiency than the nanoparticles, in which the targeting moieties are covalently bound to the nanoparticles.

Accordingly, the object underlying the present invention is to provide a modified nanoparticle with one or more targeting moieties attached on the surface thereof, which has a high and preferably improved targeting efficiency in blood plasma and blood and preferably also a high cellular uptake, i.e. to provide a nanoparticle in which the one or more targeting moieties are neither removed nor masked after the formation of protein corona when the modified nanoparticle is contacted with blood plasma or blood, and which is easy to prepare.

In accordance with the present invention, this object is satisfied by providing a modified nanoparticle for use in a therapeutic method, wherein the therapeutic method comprises the administration of the modified nanoparticle to an organism, and wherein the modified nanoparticle is obtainable by a process comprising the steps of i) providing a nanoparticle and ii) contacting the nanoparticle with one or more targeting moieties at a pH value of less than 7.0 so as to non-covalently bind the one or more targeting moieties onto the surface of the nanoparticle, wherein the nanoparticle provided in step i) is made of a material having at least one protonable or deprotonable group on the surface thereof and/or the one or more targeting moieties contacted with the nanoparticle in step ii) has at least one protonable or deprotonable group.

This solution bases on the surprising finding that by non-covalently binding a targeting moiety onto the surface of a nanoparticle - wherein the nanoparticle is made of a material having at least one protonable or deprotonable group on the surface thereof and/or the one or more targeting moieties contacted with the nanoparticle in step ii) has at least one protonable or deprotonable group - at a specific, namely acidic pH value, a modified nanoparticle is obtained, in which the targeting moiety is not only strongly bound to the surface of the nanoparticle, but which has an improved targeting efficiency and a high cellular uptake. In particular, the targeting moiety is neither notably removed nor notably masked after the formation of protein corona when the modified nanoparticle in accordance with the present invention is contacted with blood plasma or blood. It has been found by the inventors of the present invention that the targeting efficiency of the modified nanoparticle in accordance with the present invention is higher than that of a respective nanoparticle, in which the targeting moiety is covalently bound to the nanoparticle, because the protein corona significantly less negatively affects the bound targeting moiety by replacing it and/or by masking it. In addition, it has been found by the inventors of the present invention that the targeting efficiency of the modified nanoparticle in accordance with the present invention is drastically higher than that of a respective nanoparticle, in which the targeting moiety is non-covalently bound to the nanoparticle at a physiological pH value of 7.35 to 7.45. In particular, experiments of the inventors of the present invention have shown that by non-covalently binding the targeting moiety to the nanoparticle at a physiological pH value of 7.35 to 7.45, only a weak or even no binding is obtained or the binding is not stable, i.e. the targeting moieties detach from the nanoparticle, once the modified nanoparticle is contacted with biological fluid.

Without wishing to be bound to any particular theory, it is considered by the inventors that one of the reasons for these surprising effects and improvements is that by binding the targeting moiety at the optimized acidic pH value the targeting moiety, i.e. a protein, particularly an antibody, or a complex sugar, at least partially unfolds (i.e. denatures) and exposes hydrophobic residues of the targeting moiety, which allows that the targeting moiety is strongly bound to the nanoparticle at least partially by adsorption or hydrophobic interactions, respectively, and/or electrostatic interactions and which further allows that the part of the targeting moiety being responsible for recognizing a specific cell marker is exposed on the surface end of the bound targeting moiety, i.e. the end of the bound moiety being opposite to the surface of the nanoparticle. More specifically, it is considered that by binding an antibody as the targeting moiety at the optimized acidic pH value the $C_H2$-domain of the antibody which located within the Fc region of the antibody at least partially unfolds and thereby exposes hydrophobic residues, which interact with the surface of the nanoparticles. Since the antibody is bound via the Fc region, the Fab regions of the antibody are oriented to and exposed on the surface end of the bound targeting moiety. On account of these reasons, the part of the targeting moiety, which is responsible for the targeting function, is after the formation of the protein corona after contacting the modified nanoparticle with blood or blood plasma more accessible than that of a nanoparticle, in which the targeting moieties are covalently bound to the nanoparticle. All in all, the modified nanoparticle in accordance with the present invention remains during its use functional and is not completely exchanged or covered by the biomolecular protein corona. Finally, the modified nanoparticle in accordance with the present invention can be easily, flexible and efficiently produced.

A nanoparticle is in accordance with the present invention a particle, whose largest dimension is at most 1,000 nm.

Moreover, targeting moiety means in accordance with the present invention a chemical moiety, group or residue, respectively, which facilitates the transport of a nanoparticle or other carrier, to which the targeting moiety is bound, to a particular targeting site, such as a specific cell type, a specific tissue or a specific organ, and which is able to bind to or otherwise associate at the targeting site with a biological moiety, for example with a membrane component or a cell surface receptor. Therefore, the targeting moiety is in particular a cell-targeting moiety.

In accordance with the present invention, the targeting moiety is non-covalently bound to the surface of the nanoparticle before the so obtained modified nanoparticle is administered to an organism, i.e. into a biological fluid, such as blood, of the organism. In other words, the targeting moiety of the modified nanoparticle in accordance with the present invention does not derive from the protein corona formed after contacting the modified nanoparticle with the biological fluid after administration of the nanoparticle to the organism, but has been non-covalently bound to the nanoparticle prior to the administration.

According to the present invention, the targeting moiety is non-covalently bound to the surface of the nanoparticle. Without wished to be bound by any theory, it is considered by the inventors that the non-covalent bonds are at least partially adsorptive bonds, such as hydrophobic interactions, and/or electrostatic interactions. The modified nanoparticles in accordance with the present invention preferably do not contain (additional) target moieties being covalently bound to the nanoparticle, i.e. preferably all target moieties are non-covalently bound to the nanoparticle. However, even if this is less preferred in accordance with the present invention, it might be possible, such as in the case of a wished dual-targeting, that a part of the targeting moiety molecules (such as one kind of the two different targeting moiety molecules) may be covalently bound to the nanoparticle, whereas the remaining part is non-covalently bound.

The modified nanoparticle of the present invention is for use in a therapeutic method, which comprises the targeting of the modified nanoparticles to a specific site in the organism preferably followed by an uptake of the modified nanoparticle into a cell. This allows the modified nanoparticle to act as drug delivery system. For this purpose, it is preferred that the modified nanoparticle is filled with one or more drugs and/or that one or more drugs are bound to the surface of the nanoparticle, either covalently and/or non-covalently, and/or the drug is otherwise transported together with the nanoparticle. The first mentioned alternative of being filled with one or more drugs may be accomplished by using hollow nanoparticles, such as nanotubes or nanocapsules.

In accordance with a first particularly preferred embodiment, the nanoparticle provided in step i) has at least one deprotonable group on the surface thereof. The present invention is not particularly limited concerning the chemical nature of the deprotonable group, as long as the group contains a hydrogen atom and as long as a proton may be abstracted or partly abstracted from this group in an aqueous medium at a pH value of less than 7.0. The nanoparticle provided in step i) may have only one kind of deprotonable group, such as a carboxy group, or two or more different kinds of deprotonable groups, such as a carboxy group and a hydroxyl group. However, it is preferred in this embodiment that only deprotonable group(s) and no protonable groups are bound to the surface of the nanoparticle. Of course, the nanoparticle will have a plurality of single respective groups on its surface, such as for instance at least one such group per 10 square nanometer of the surface of the nanoparticle, at least one such group per 5 square nanometer of the surface of the nanoparticle, at least one such group per 2 square nanometer of the surface of the nanoparticle, at least one such group per 1 square nanometer of the surface of the nanoparticle or at least two such groups per 1 square nanometer of the surface of the nanoparticle.

Good results are in particular obtained, when the at least one deprotonable group is selected from the group consisting of carboxy groups, hydroxyl groups, thiol groups, sulfenic acid groups, sulfinic acid groups, sulfonic acid groups, thiocarboxylic groups, peroxy carboxylic groups, oxime groups and arbitrary combinations of two or more of the aforementioned groups.

More preferably, the at least one deprotonable group is selected from the group consisting of carboxy groups and/or hydroxyl groups.

Particular good results are obtained, when the nanoparticle is made of a polymer - like a polystyrene, a polyacrylate, a polymethacrylate, a poly-L-lactic acid or a poly-L-lactic acid-co-glycolic acid - which can be surface modified with carboxy groups, or when the nanoparticle is made of a protein, like albumin, or made of a polyamino acid, such as poly-L-lysine, or made of hydroxyethyl starch. A polystyrene being surface modified with carboxy groups may be obtained by copolymerizing styrene and (methacrylic) acid, wherein both monomers may be mixed before starting the polymerization, or, wherein first styrene is partially polymerized and then the (methacrylic) acid is added and copolymerization is continued.

Most preferably, the nanoparticle provided in step i) is made of hydroxyethyl starch or of a protein, like albumin. Apart from having a high density of hydroxyl groups on the surface thereof, the material is biodegradable and physiologically well compatible.

In accordance with the present invention, the nanoparticle provided in step i) is contacted with the one or more targeting moieties in step ii) at a pH value of less than 7.0, preferably at a pH value of at most 6.5 and more preferably at a pH value of 1.0 to 6.5. The exact optimal pH value depends on the kind of the surface groups of the nanoparticle, i.e. on the chemical nature of protonable or deprotonable groups, and on the chemical nature of the at least one targeting moiety. For each pair of nanoparticle/ targeting moiety an optimal acidic pH exists, which can be easily found by performing a pH dependent test series and then determining the targeting efficiency of the modified nanoparticles as described in more detail below. The inventors have tested a plurality of pairs of nanoparticles with deprotonable groups and antibodies as targeting moieties and in particular pairs of nanoparticles with carboxy groups and/or hydroxyl groups (in particular polystyrene which is surface modified with carboxyl groups and hydroxyethyl starch) and antibodies as targeting moieties and have found two distinct pH value ranges.

Accordingly, in accordance with a first particular preferred variant of this embodiment of the present invention, in which the nanoparticle has deprotonable groups on its surface, the optimal pH value, at which the nanoparticle is contacted in step ii) with the one or more targeting moieties, is between 1.0 and 4.2, preferably between 1.7 and 3.7, more preferably between 2.1 and 3.3, yet more preferably between 2.4 and 3.0, still more preferably between 2.6 and 2.8 and most preferably at about 2.7. This embodiment is particularly preferred for nanoparticles made of hydroxyethyl starch.

In accordance with a second particular preferred variant of this embodiment of the present invention, the optimal pH value, at which the nanoparticle is contacted in step ii) with the one or more targeting moieties between 5.0 and 6.5, more preferably between 5.5 and 6.5, even more preferably between 5.8 and 6.4, yet more preferably between 5.9 and 6.3, still more preferably between 6.0 and 6.2 and most preferably at about 6.1. This embodiment is particularly preferred for nanoparticles made of polystyrene, which is surface modified with carboxyl groups.

In accordance with a second particularly preferred embodiment, the nanoparticle provided in step i) has at least one protonable group on the surface thereof.

The present invention is not particularly limited concerning the chemical nature of the protonable group, as long as the group contains a hydrogen atom and as long as a proton may be bound by this group in an aqueous medium at a pH value of less than 7.0. The nanoparticle provided in step i) may have only one kind of protonable group, such as an amino group, or two or more different kinds of protonable groups, such as an amino group and an amide group. However, it is preferred in this embodiment that only protonable group(s) and no deprotonable groups are bound to the surface of the nanoparticle. Of course, the nanoparticle will have a plurality of single respective groups on its surface, such as for instance at least one such group per 10 square nanometer of the surface of the nanoparticle, at least one such group per 5 square nanometer of the surface of the nanoparticle, at least one such group per 2 square nanometer of the surface of the nanoparticle, at least one such group per 1 square nanometer of the surface of the nanoparticle or at least two such groups per 1 square nanometer of the surface of the nanoparticle.

Good results are in particular obtained, when the at least one protonable group is selected from the group consisting of amino groups, hydrazine groups, hydrazide groups, carboxy hydrazide groups, sulfonic acid amide groups, amide groups and arbitrary combinations of two or more of the aforementioned groups. Good results are in particular obtained with amino groups and/or amide groups. Therefore, it is particularly preferred that the nanoparticle is made of polystyrene, which is surface modified with amino groups and/or amide groups, or of a protein or of a polyamino acid, like poly-L-lysine. Polystyrene being surface modified with amino groups may be obtained by copolymerizing styrene and 2-aminoethyl methacrylate hydrochloride (AEMH), wherein both monomers may be mixed before starting the polymerization, or, wherein first styrene is partially polymerized and then the AEMH is added and copolymerization is continued.

As set out above, the optimal pH value for step ii) depends on the chemical nature of the surface group(s) of the nanoparticle and on the chemical nature of the at least one targeting moiety and has to be determined for each pair of nanoparticle/ targeting moiety. The inventors have tested a plurality of pairs of nanoparticles with protonable groups and antibodies as targeting moieties and in particular pairs of nanoparticles with amino groups and/or amide groups (in particular polystyrene which is surface modified with amino groups or amide groups) and antibodies as targeting moieties and have found one distinct pH value range. Therefore, it is preferred in this embodiment, in which the nanoparticle has protonable groups on its surface, that the nanoparticle is contacted with the one or more targeting moieties in step ii) at a pH value of at most 6.5, more preferably at a pH value of 1.0 to 6.5, even more preferably at a pH value of 1.0 to 4.2, yet more preferably at a pH value of 1.7 to 3.7, still preferably at a pH value of 2.1 to 3.3, yet more preferably at a pH value of 2.4 to 3.0, still more preferably at a pH value of 2.6 to 2.8 and most preferably at a pH value of about 2.7.

In accordance with a third particularly preferred embodiment, the one or more targeting moieties contacted with the nanoparticle in step ii) has at least one protonable group. The chemical nature of the protonable group, the pH value in step ii) and the other preferred features for this embodiment are likewise to those mentioned above with regard to the first particularly preferred embodiment.

In accordance with a fourth particularly preferred embodiment, the one or more targeting moieties contacted with the nanoparticle in step ii) has at least one deprotonable group. The chemical nature of the deprotonable group, the pH value in step ii) and the other preferred features for this embodiment are likewise to those mentioned above with regard to the second particularly preferred embodiment.

The present invention is not particularly limited concerning the form and dimensions of the nanoparticles, as long as the nanoparticles are able to circulate in a blood vessel. Thus, the nanoparticle may have a spherical form, a tubular form or an irregular form, wherein the largest dimension of the nanoparticle is 1 to 1,000 nm and preferably 20 to 500 nm, and wherein the smallest dimension is preferably at least 1 nm.

The present invention is also not particularly limited concerning the chemical nature of the one or more targeting moieties of the modified nanoparticle. Preferably, the at least one targeting moiety is selected from the group consisting of proteins, ribonucleic acids and/or sugars. More preferably, at least one targeting moiety is one or more antibodies, which preferably recognizes a protein or another antigen on the surface of the target cell, wherein the recognized protein or other antigen is more preferably specific for the target cell, i.e. is present on the specific target cell type, but not on other cells or at least not on any other cell of the organism.

Particular suitable examples for such targeting moieties are antibodies, such as those derived from mouse, rat, hamster or rabbit which preferably are then humanized, or hormones and/or ligands to cell surface receptors, like interleukin-2. Antibodies or ligands targeting even specific uptake and processing routes - like CD63 for dendritic cells - maybe deliberately chosen. Also, arbitrary combinations of two or more of the aforementioned compounds can be chosen.

The targeting moiety is preferably selective for one or more specific cell types, such as T-cells, monocyte derived dendritic cells, or other cells of the immune system, the vasculature or organ or malignant cells.

The nanoparticle obtained in step ii) may have only one kind of targeting moiety or two or more different kinds of targeting moieties bound thereon. Of course, the nanoparticle will have a plurality of single respective targeting moiety molecules non-covalently bound on its surface, such as for instance at least one such group per 10 square nanometer of the surface of the nanoparticle, at least one such group per 5 square nanometer of the surface of the nanoparticle, at least one such group per 2 square nanometer of the surface of the nanoparticle, at least one such group per 1 square nanometer of the surface of the nanoparticle or at least two such groups per 1 square nanometer of the surface of the nanoparticle.

In a further development of the idea of the present invention, it is proposed that the modified nanoparticle in accordance with the present invention does not comprise any targeting moiety, which is covalently bound to the material of the nanoparticle. However, even if this is less preferred in accordance with the present invention, it might be possible, such as in the case of a wished dual-targeting, that a part of the targeting moiety molecules (such as one kind of the two different targeting moiety molecules) may be covalently bound to the nanoparticle, whereas the remaining part is non-covalently bound.

Preferably, the modified nanoparticle in accordance with the present invention is in vivo targeted to a specific site of the organism, to which is has been administered in the therapeutic method. It is in particular preferred that the therapeutic method comprises drug delivery. In this embodiment, the nanoparticle is preferably filled with a drug or a drug is bound to the surface of the nanoparticles. This could be any drug desired to be delivered by a nanocarrier, more specifically a messenger ribonucleic acid (mRNA), a small interfering RNA (siRNA), a desoxyribonucleic acid (DNA) or any other kind of nucleic acid, as well as a protein, peptide or small chemical organic or inorganic compound.

The modified nanoparticle can be administered to the organism in the form as obtained in step ii) or, alternatively, the pH of the modified nanoparticle obtained in step ii) may be adjusted in an optional step iii) to the physiological pH value, which is between 7.35 and 7.45 and thus about 7.4.

The present invention is not particularly limited concerning to the organism, to which the modified nanoparticle is administered. Particular suitable as organism is a vertebrate, a mammalian or a human being.

Preferably, the modified nanoparticle is administered in the therapeutic method to a biological fluid of the organism and preferably to the blood.

The modified nanoparticle may be administered to the organism intraarterially, intravenously, intramuscularly, intrathecally, dermally, inhaled or orally.

According to a further subject matter, the present invention relates to a modified nanoparticle for use in a therapeutic method, wherein the method comprises the steps of: i) providing a nanoparticle of a material having functional groups with a protonable or deprotonable group on the surface thereof, ii) contacting the nanoparticle with one or more targeting moieties at a pH value of less than 7.0 so as to non-covalently bind the one or more targeting moieties onto the surface of the nanoparticle and iii) administering the so obtained modified nanoparticle to an organism.

In addition, the present invention relates to the non-medical use of a modified nanoparticle, wherein the modified nanoparticle is contacted in vitro with a fluid, preferably with a biological fluid, more preferably with a protein containing biological fluid, a lipid containing fluid or a sugar containing biological fluid, and wherein the modified nanoparticle is obtainable by a process comprising the steps of i) providing a nanoparticle and ii) contacting the nanoparticle with one or more targeting moieties at a pH value of less than 7.0 so as to non-covalently bind the one or more targeting moieties onto the surface of the nanoparticle, wherein the nanoparticle provided in step i) is made of a material having at least one protonable or deprotonable group on the surface thereof and/or the one or more targeting moieties contacted with the nanoparticle in step ii) has at least one protonable or deprotonable group. For instance, the fluid may be water or an aqueous solution and the modified nanoparticle is used for plant protection.

If a biological fluid is used, with which the modified nanoparticle is contacted, it may be any protein containing fluid, such as particularly blood, blood plasma or blood serum derived from an organism, such as a vertebrate, a mammalian or a human being.

The purpose of the non-medical use may be to determine in vitro the targeting efficiency of the modified nanoparticle to a specific site, such as to a specific cell type. Alternatively, each other use is possible, in which a nanoparticle has to reach its target through a medium, such as in particular through a protein containing medium. For instance, a modified nanoparticle may be used to bind bacteria in sewage sludge, whereafter the nanoparticle is destroyed or removed, such as magnetically removed, from the sludge.

Furthermore, the present invention is directed to a method for preparing a modified nanoparticle, which comprises the steps of i) providing a nanoparticle and ii) contacting the nanoparticle with one or more targeting moieties at a pH value of less than 7.0 so as to non-covalently bind the one or more targeting moieties onto the surface of the nanoparticle, wherein the nanoparticle provided in step i) is made of a material having at least one protonable or deprotonable group on the surface thereof and/or the one or more targeting moieties contacted with the nanoparticle in step ii) has at least one protonable or deprotonable group.

The aforementioned preferred embodiments, in particular concerning the chemical nature of the at least one protonable or deprotonable group, the pH value adjusted in step ii) and the kind of nanoparticle, are also preferred for the method for preparing a modified nanoparticle in accordance with the present invention.

Moreover, the present invention is related to a modified nanoparticle obtainable by a method comprising the steps of i) providing a nanoparticle and ii) contacting the nanoparticle with one or more targeting moieties at a pH value of less than 7.0 so as to non-covalently bind the one or more targeting moieties onto the surface of the nanoparticle, wherein the nanoparticle provided in step i) is made of a material having at least one protonable or deprotonable group on the surface thereof and/or the one or more targeting moieties contacted with the nanoparticle in step ii) has at least one protonable or deprotonable group.

Subsequently, the present invention is further described by means of illustrative, but not limiting examples and figures.

Fig. 1a      shows the binding properties of the targeting moiety molecules of the modified nanoparticles of two references, of comparative example 1 and of example 1 as described below as measured fluorescence, namely as the amount of Alexa Fluor 647 positive nanoparticles in %.

Fig. 1b      shows the binding properties of the targeting moiety molecules determeined by western blot analysis of the modified nanoparticles of two references, of comparative example 1 and of example 1 as described below.

Fig. 2       shows the binding properties of the targeting moiety molecules in the presence of protein corona by means measured fluorescence of the modified nanoparticles of two references, of comparative example 1 and of example 1 as described below.

Fig. 3       shows the measured cell uptake or cell adhesion of the modified nanoparticles of two references, of comparative example 1 and of example 1 as described below.

Fig. 4       shows the measured cell uptake or cell adhesion in the presence of protein corona with cells with unblocked or blocked FcR receptors of the modified nanoparticles of two references, of comparative example 1 and of example 1 as described below.

Fig. 5a      shows the cellular uptake or cell adhesion of nanoparticles of polystyrene functionalized with carboxy groups and with targeting moiety molecules obtained at different pH values in cells in 0% human serum as described below in example 3.

Fig. 5b      shows the cellular uptake or cell adhesion of nanoparticles of polystyrene functionalized with carboxy groups and with targeting moiety molecules obtained at different pH values in cells in 0% human serum as described below in example 3.

Fig. 6a      shows the cellular uptake or cell adhesion of nanoparticles of polystyrene functionalized with amino groups and with non-covalently bound targeting moiety molecules obtained at different pH values in cells in 0% human serum as described below in example 4.

Fig. 6b      shows the cellular uptake or cell adhesion of nanoparticles of polystyrene functionalized with amino groups and with non-covalently bound targeting moiety molecules obtained at different pH values in cells in 10% human serum as described below in example 4.

Fig. 7a      shows the amount of non-covalently bound antibody on nanoparticles of hydroxyethyl starch obtained at different pH values as described below in example 5.

Fig. 7b      shows the cellular uptake or cell adhesion of nanoparticles of hydroxyethyl starch with non-covalently bound antibody obtained at different pH values in cells as described below in example 5.

**Example 1 - Nanoparticles modified with carboxy groups and with anti-CD63 non-covalently bound as targeting moiety**

(*Synthesis of oleate-capped iron oxide nanoparticles*)

Hydrophobic oleate-stabilized iron oxide nanoparticles were prepared as described by Bannwarth et al., "Colloidal Polymers with Controlled Sequence and Branching Constructed from Magnetic Field Assembled Nanoparticles" in ACS Nano 2015, volume 9, pages 2720 to 2728. More specifically, 60 mmol of $FeCl_2$ - 4 $H_2O$ and 90 mmol of $FeCl_3$ - 6 $H_2O$ were mixed in 100 mL of deionized water and were mechanically stirred. 40 mL of concentrated ammonium hydroxide solution were added drop-wise over 5 min at room temperature. Afterwards, 5.0 g of oleic acid were added and the mixture was heated to 70 °C with constant stirring. After 1 h, the temperature was raised to 110 °C and the reaction continued for 2 h, keeping the volume constant by adding water sporadically. The resulting black precipitate was washed several times with water and dried overnight under vacuum at 40 °C.

(*Synthesis of magnetic polystyrene nanoparticles functionalized with carboxylic acid groups*)

Magnetic polystyrene nanoparticles functionalized with carboxylic acid groups were prepared as described in the aforementioned publication of Bannwarth et al. with the following minor modifications so as to obtain magneto-responsive polystyrene nanoparticles labelled with a fluorescent dye and functionalized with carboxylic acid groups. First, 1.0 g of the aforementioned iron oxide nanoparticles were mixed with 1.5 mg of the boron-dipyrromethene dye (BODIPY) and dispersed in 0.5 g of octane in an ultrasonication bath during 30 min. Then, 24 mL of an aqueous solution of sodium dodecyl sulphate (2.5 mg/mL; SDS) were added and the system was emulsified by ultrasonication with a tip sonifier for 3 min (70% amplitude, 5 s pulse, 5 s pause) under ice cooling. The obtained mini-emulsion was transferred to a two-necked round bottom flask and stirred mechanically at room temperature. Separately, 1.2 g of styrene were mixed with 20 mg of hexadecane and 24 mL of an aqueous SDS solution (1.0 mg/mL). The mixture was then stirred at 1,000 rpm for 1 h, ultrasonicated with a tip sonifier (1 min, 10% amplitude, 5 s pulse, 5 s pause) and mixed with the initial iron oxide-containing miniemulsion. Argon was bubbled for 10 min through the reaction mixture and after the addition of 35 mg of potassium persulfate, the system was heated to 80 °C during mechanical stirring. After 30 min, 50 mg of acrylic acid was added and the reaction proceeded for further 14 h. After cooling down, the particles were washed and purified several times with water using a permanent magnet and discarding the supernatant. The precipitate was dispersed in 1.0 mL of an aqueous SDS solution (0.2 wt%) and diluted with 6.0 mL of water.

The sample was subjected to a single centrifugation step (Sigma 3k30, 45 g, 7 min) and the supernatant was collected and characterized as follows:

## hydrodynamic diameter (Dh) = 230 ± 140 nm,

solid content = 2.9 wt%,

inorganic content 52 wt% determined by thermogravimetric analysis (TGA),

Mw = 42,000 g/mol as determined by GPC against a polystyrene standard.

The characterization has been performed as follows: The average hydrodynamic diameter of the particles was determined by dynamic light scattering (DLS) at 20 °C at a fixed angle of 90° with a PSS Nicomp particle sizer (380 Nicomp Particle Sizing Systems, USA). TGA measurements were performed on a Mettler-Toledo 851 (Mettler-Toledo, USA) thermobalance with temperatures ranging from 35 °C to 1000 °C at a heating rate of 10 °C/min under nitrogen flow (30 mL/min). The solid content of the dispersions was determined gravimetrically after freeze-drying of the dispersions. Gel permeation chromatography (GPC) was used to determine the molecular weight with a PSS SECcurity (Agilent Technologies 1260 Infinity (Agilent Technologies, USA). The freeze-dried samples were dissolved in THF and eluted at a flow rate of 1.0 mL/min at 30 °C. Polystyrene standards were used for calibration.

The nanoparticles were diluted to a concentration of 5 mg/mL in MES buffer (50 mM, pH 6.1 adjusted with NaOH 0.1 M) so as to obtain a suspension of magnetic polystyrene nanoparticles functionalized with carboxylic acid groups.

(*Non-covalently binding a targeting moiety to the nanoparticles*)

100 $\mu$L of the aforementioned suspension of magnetic polystyrene nanoparticles labelled with BODIPY and functionalized with carboxylic acid groups were diluted with 4 mL of MES buffer adjusted to pH 6.1. This diluted dispersion was then poured drop-wise onto a solution of an antibody, namely 100 $\mu$L of anti-CD63 (1.0 mg/mL) (Biolegend) dissolved in 4 mL MES buffer adjusted to pH 6.1. The mixture was incubated at room temperature for 4 h, washed twice by magnetic purification, and diluted to a final volume of 2 mL with water.

(*Determining the binding properties of the non-covalently bound targeting moieties*)

Anti-CD63 antibody molecules were detected on the surfaces of the nanoparticles by a fluorescently (Alexa Fluor 647) labelled, secondary anti-IgG1 antibody against the Fc region of the antibody or targeting moiety, respectively.

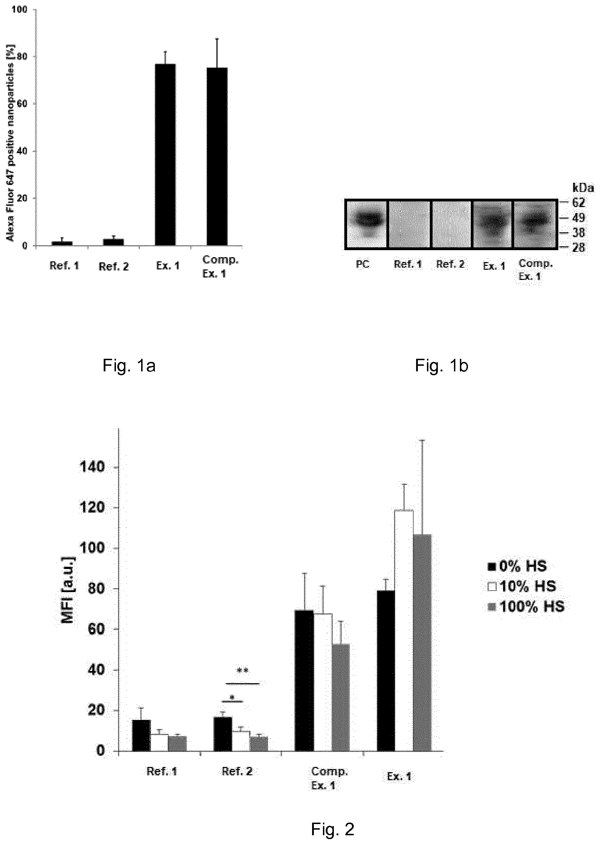

The results are shown in figure 1a, in which the measured fluorescence is shown for different samples. Data are shown as the amount of Alexa Fluor 647 positive nanoparticles in % and is the mean $\pm$ standard deviation (s.d.) of three independent experiments.

In figure 1a, Ref. 1 is a first reference, in which nanoparticles with carboxy groups prepared as described above in example 1, but without binding any targeting moiety have been used. Ref. 2 is a second reference, in which nanoparticles activated with EDC/NHs as described below with regard to comparative example 1 without binding any targeting moiety have been used. Ex. 1 has been performed with the nanoparticles obtained in example 1 with non-covalently bound targeting moiety molecules and Comp. Ex. 1 has been performed with the nanoparticles obtained in the below described comparative example 1 with covalently bound targeting moiety molecules.

As shown in figure 1a, similar amounts of anti-CD63 antibodies were detected on the nanoparticles of example 1, in which the targeting moiety molecules were non-covalently bound, as for the below described comparative example 1, in which the targeting moiety molecules were covalently bound to the nanoparticle. As expected, in both references 1 and 2, in which nanoparticles were used without any bound targeting residues, no targeting moieties could be detected.

In addition, it was tested by western blot analysis using anti-CD63 as positive control (PC) if the antibodies present as targeting moiety molecules on the surface of the nanoparticles could still bind to their target, the CD63 antigen on the monocyte derived dendritic cell (moDC). The results are shown in figure 1b. In figure 1b, the legends Ref. 1, Ref. 2, Ex. 1 and Comp. Ex. 1 are those as described above for figure 1a. PC is the positive control. As shown in figure 1b, similar amounts of anti-CD63 antibodies were detected on the nanoparticles of example 1, in which the targeting moiety molecules were non-covalently bound, as for the below described comparative example 1, in which the targeting moiety molecules were covalently bound to the nanoparticle.

(*Determining the binding properties of the non-covalently bound targeting moieties in the presence of protein corona*)

The modified nanoparticles of example 1 with the non-covalently bound targeting moiety molecules has been analyzed in the presence of protein corona. More specifically, the targeting moiety molecules bound on the surface of the nanoparticles have been detected in the presence of protein corona. Therefore, 0.4 $\mu$g nanoparticles were incubated with different concentrations of human serum (HS), namely with 0% HS, 10% HS and 100% HS, for 2 h at 37 °C to allow protein adsorption, i.e. the formation of protein corona on the modified nanoparticles. Fluorescent secondary mouse antibodies against mouse IgG1 were used to investigate the accessibility of the antibody on the nanoparticle surface after corona formation. The median fluorescence intensity (MFI) was determined via flow cytometry.

The results are shown in figure 2. In figure 2, the legends Ref. 1, Ref. 2, Ex. 1 and Comp. Ex. 1 are those as described above for figure 1a. As shown in figure 2, the formation of protein corona on the modified nanoparticle of example 1, in which the targeting moiety molecules are non-covalently bound, does not at all reduce the accessibility of the targeting moiety, since the measured fluorescence intensities for the samples in 10% and 100% HS are higher than the measured fluorescence intensity of the sample in 0% HS. In contrast thereto, in comparative example 1, in which the targeting moiety molecules are covalently bound to the nanoparticles, the presence of protein corona negatively affects the accessibility of the targeting moiety, as it derives from the fact the measured fluorescence intensities for the samples in 10% and 100% HS are lower than the measured fluorescence intensity of the sample in 0% HS. As expected, for the controls Ref. 1 and Ref. 2, in which no targeting moiety molecules are bound to the nanoparticles, no significant values are obtained.

(*Cellular uptake or cell adhesion in cell culture medium*)

The cellular uptake of the modified nanoparticles obtained in this example has been determined as follows: Monocyte derived dendritic cells (moDCs) were generated from healthy human donor buffy coats which were obtained according to the vote of the local ethics committee and the Declaration of Helsinki. Isolation of peripheral blood mononuclear cells (PBMCs) was performed by standard Ficoll separation. $1.5 \cdot 10^7$ PBMCs per well of 6-well plates were allowed to attach in 3 mL DC-medium containing RPMI-1640 (Sigma-Aldrich Chemie GmbH) with 2% human serum (HS, Lonza), 100 IU/mL penicillin and 100 $\mu$g/mL streptomycin (Life Technologies GmbH). After 1 h incubation under standard conditions (37 °C, 5% $CO_2$) non-adherent cells were discarded by washing the 6-well plates with phosphate buffered saline (PBS, Sigma-Aldrich). To generate moDCs, monocytes were incubated for 48 h in 3 mL DC-medium with 800 IU/mL granulocyte-macrophage colony-stimulating factor (GMCSF, Sanofi-Aventis) and 500 IU/mL interleukin IL4 (PromoCell GmbH). 800 $\mu$L of supernatant were centrifuged and replaced by 1 mL DC-medium with 1600 IU/mL GMCSF and 500 IU/mL IL4 followed by 72 h incubation under standard conditions. This procedure was repeated and cells were incubated for a further 24 h. MoDCs were released from 6-well plates by using PBS, 0.5 mM ethylenediaminetetraacetic acid (EDTA).

For determining the nanoparticle uptake or cell adhesion in moDCs, around 100,000 moDCs in 200 $\mu$L DC-medium with 1600 IU/mL GMCSF and 500 IU/mL IL4 were seeded into the wells of a 24-well plate. The cells were incubated at different nanoparticle concentrations (1.875, 3.75, 7.5 and 10 $\mu$g/mL) for 2 h under standard conditions. Then, the cellular uptake or cell adhesion was analyzed by flow cytometry.

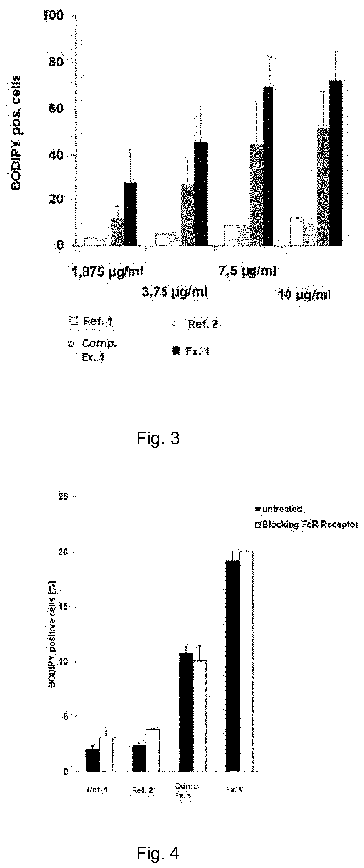

The results are shown in figure 3. The values are shown as the amount of nanoparticle (BODIPY) positive cells in % and are given as mean $\pm$ s.d. of three independent experiments. In figure 3, the legends Ref. 1, Ref. 2, Ex. 1 and Comp. Ex. 1 are those as described above for figure 1a.

As shown in figure 3, the cellular uptake or cell adhesion of the nanoparticles of example 1 in accordance with the present invention is at any of the tested concentrations significantly higher as the cellular uptake or cell adhesion of the nanoparticles of comparative example 1, in which the targeting moiety molecules are covalently bound, and drastically higher as in the controls Ref. 1 and Ref. 2, in which no targeting moiety is bound to the nanoparticle.

(*Cellular uptake or cell adhesion in the presence of protein corona and with cells with blocked FcR receptors*)

The cellular uptake or cell adhesion of the modified nanoparticles obtained in this example has been determined as described above, but not in cell culture medium, but in the presence of protein corona with cells, in which the FcR receptors have been blocked. More specifically, the moDCs were pre-incubated with FcR blocking solution according to the manufactures instruction (Biolegend). The modified nanoparticles (5 $\mu$g/mL) of example 1 were incubated with 100% HS for 2 h, 37°C and subsequently added to the moDCs for 2 h at 4 °C. The blocking solution was kept in the cell culture medium to ensure an efficient blocking of the FcR receptors during the incubation of nanoparticles and cells.

As reference, the cellular uptake or cell adhesion of the modified nanoparticles of example 1 was evaluated as described above, but without blocking of the FcR receptors of the moDCs.

The results are shown in figure 4. The values are shown as the amount of nanoparticle (BODIPY) positive cells in % and given as mean ± s.d. of three independent experiments. In figure 4, the legends Ref. 1, Ref. 2, Ex. 1 and Comp. Ex. 1 are those as described above for figure 1a.

As shown in figure 4, the cellular uptake or cell adhesion of the modified nanoparticles of example 1 in accordance with the present invention is not diminished by the protein corona, even when the FcR receptors of the cells have been blocked. Rather, the values obtained with the protocol performed with moDCs with blocked FcR receptors (white column in Ex. 1 of figure 4) is even slightly higher than those obtained with the protocol performed with moDCs without blocked FcR receptors (black column in Ex. 1 of figure 4). This shows that the protein corona does not negatively influence the cellular uptake or cell adhesion of the modified nanoparticles of the present invention and that indeed the targeting and uptake of the modified nanoparticles of the present invention is based on the antigen binding part of the antibody (i.e. the targeting moiety), which is located on the two Fab regions, and not based on any of the protein corona proteins. As further shown in figure 4, the cellular uptake or cell adhesion of the modified nanoparticles of the example 1 in accordance with the present invention, in which the targeting moiety molecules are non-covalently bound to the nanoparticles, is significantly higher than the cellular uptake or cell adhesion of the modified nanoparticles of comparative example 1, in which the targeting moiety molecules are covalently bound to the nanoparticles.

**Comparative Example 1 - Nanoparticles modified with carboxy groups and with anti-CD63 covalently bound as targeting moiety**

The method of example 1 has been repeated except that the step of non-covalently binding anti-CD63 antibody as targeting moiety to the nanoparticles has been replaced by the following steps for covalently binding anti-CD63 antibody as targeting moiety to the nanoparticles.

The covalent attachment of the anti-CD63 antibodies to the surface of the magnetic nanoparticles was carried out by EDC (1-ethyl-3-(3-dimethylaminopropyl)carbodiimide) NHS (N-hydroxysuccinimide) coupling, which is a two-step process that involves the activation of a carboxyl group and subsequent conjugation with a primary amine.

(*Activation*)

To 1 mL of the suspension of magnetic polystyrene nanoparticles functionalized with carboxylic acid groups 17.26 mg (0.15 mmol) of NHS and 5.75 mg (0.03 mmol) of EDC-HCl were added and the mixture was shaken for 1 h at room temperature. The reaction mixture was later washed three times with MES buffer by magnetic purification to remove the unreacted material and diluted to a final volume of 1 mL.

(*Conjugation*)

100 μL of the dispersion of EDC/NHS-activated particles were diluted with 4 mL of MES buffer. 100 μL of anti-CD63 (1.0 mg/mL) were dissolved in 4 mL of MES buffer and the obtained mixture was added drop-wise to the antibody solution. Finally, the combined reaction mixture was shaken for 4 h at room temperature and washed five times with water by magnetic purification. The final volume of the dispersion was adjusted to 2 mL.

(*Determining the binding properties of the covalently bound targeting moieties*)

Anti-CD63 antibody molecules were detected on the surfaces of the nanoparticles by a fluorescently (Alexa Fluor 647) labelled, secondary anti-IgG1 antibody against the Fc region of the antibody or targeting moiety, respectively. The results are shown in figure 1a.

In figure 1a, the legends Ref. 1, Ref. 2, Ex. 1 and Comp. Ex. 1 are those as described above for example 1. As shown in figure 1a, similar amounts of anti-CD63 antibodies were detected on the nanoparticles of comparative example 1, in which the targeting moiety molecules were covalently bound, as for the above described example 1, in which the targeting moiety molecules were non-covalently bound to the nanoparticle.

In addition, it was tested by western blot analysis using anti-CD63 as positive control (PC) if the antibodies present as targeting moiety molecules on the surface of the nanoparticles could still bind to their target, the CD63 antigen on the monocyte derived dendritic cell (moDC). The results are shown in figure 1b. In figure 1b, the legends Ref. 1, Ref. 2, Ex. 1 and Comp. Ex. 1 are those as described above for figure 1a. PC is the positive control. As shown in figure 1b, similar amounts of anti-CD63 antibodies were detected on the nanoparticles of example 1, in which the targeting moiety molecules were non-covalently bound, as for the below described comparative example 1, in which the targeting moiety molecules were covalently bound to the nanoparticle.

*(Determining the binding properties of the covalently bound targeting moieties in the presence of protein corona)*

The modified nanoparticles of comparative example 1 with the covalently bound targeting moiety molecules has been analyzed as described above with regard to example 1.

The results are shown in figure 2. In figure 2, the legends Ref. 1, Ref. 2, Ex. 1 and Comp. Ex. 1 are those as described above for figure 1a.

As shown in figure 2, in comparative example 1, in which the targeting moiety molecules are covalently bound to the nanoparticles, the presence of protein corona negatively affects the accessibility of the targeting moiety, as it derives from the fact the measured fluorescence intensities for the samples in 10% and 100% HS are lower than the measured fluorescence intensity of the sample in 0% HS. In contrast thereto, the formation of protein corona on the modified nanoparticle of example 1, in which the targeting moiety molecules are non-covalently bound, does not at all reduce the accessibility of the targeting moiety, since the measured fluorescence intensities for the samples in 10% and 100% HS are higher than the measured fluorescence intensity of the sample in 0% HS.

*(Cellular uptake or cell adhesion in cell culture medium)*

The cellular uptake or cell adhesion of the modified nanoparticles obtained in this comparative example was determined as described with regard to example 1 above.

The results are shown in figure 3. In figure 3, the legends Ref. 1, Ref. 2, Ex. 1 and Comp. Ex. 1 are those as described above for figure 1a. As shown in figure 3, the cellular uptake or cell adhesion of the nanoparticles of the comparative example 1, in which the targeting moiety molecules covalently bound to the nanoparticles, is at any of the tested concentrations significantly lower than the cellular uptake or cell adhesion of the nanoparticles of example 1 in accordance with the present invention, in which the targeting moiety molecules non-covalently bound to the nanoparticles.

*(Cellular uptake or cell adhesion in the presence of protein corona and with cells with blocked FcR receptors)*

The cellular uptake or cell adhesion of the modified nanoparticles obtained in this comparative example in cells, in which the FcR receptors have been blocked, has been determined as described above for example 1.

The results are shown in figure 4. In figure 4, the legends Ref. 1, Ref. 2, Ex. 1 and Comp. Ex. 1 are those as described above for figure 1a.

As shown in figure 4, the cellular uptake or cell adhesion of the modified nanoparticles of the comparative example 1, in which the targeting moiety molecules are covalently bound to the nanoparticles, is slightly reduced in the samples with cells with blocked FcR receptors of the cells have been blocked than in the samples with unblocked cells. Thus, the protein corona has a small effect on the cellular uptake or cell adhesion of the modified nanoparticles of comparative example 1. However, the cellular uptake or cell adhesion of the modified nanoparticles of the comparative example 1 is significantly lower than the cellular uptake or cell adhesion of the modified nanoparticles of example 1, in which the targeting moiety molecules are non-covalently bound to the nanoparticles. Moreover, the cellular uptake or cell adhesion of the modified nanoparticles of example 1 in accordance with the present invention is not diminished by the protein corona, even when the FcR receptors of the cells have been blocked. Rather, the values obtained with the protocol performed with moDCs with blocked FcR receptors (white column in Ex. 1 of figure 4) is even slightly higher than those obtained with the protocol performed with moDCs without blocked FcR receptors (black column in Ex. 1 of figure 4).

**Example 2 - Nanoparticles modified with carboxy groups and with mouse IgG as targeting moiety non-covalently bound as targeting moiety**

The method of example 1 has been repeated except that in the step of non-covalently binding a targeting moiety to the nanoparticles 40 μL of mouse IgG (2.5 mg/mL) (Invitrogen) dissolved in 4 mL MES buffer adjusted to pH 6.1 have been used instead of 100 μL of anti-CD63 (1.0 mg/mL) dissolved in 4 mL MES buffer.

The properties of the non-covalently bound targeting moieties have been determined as described in example 1 and similar results have been obtained.

**Comparative Example 2 - Nanoparticles modified with carboxy groups and with mouse IgG covalently bound as targeting moiety**

The method of comparative example 1 has been repeated except that in the conjugation step 40 μL of mouse IgG (2.5 mg/mL) dissolved in 4 mL MES buffer have been used instead of 100 μL of anti-CD63 (1.0 mg/mL) dissolved in 4 mL MES buffer.

The properties of the covalently bound targeting moieties have been determined as described in comparative example 1 and similar results have been obtained.

**Example 3 - Nanoparticles modified with carboxy groups with anti-CD63 antibody or mouse IgG1 antibody non-covalently bound as targeting moiety at different pH values**

Modified nanoparticles of polystyrene, which have carboxy groups on their surface, have been non-covalently coupled with anti-CD63 antibody or with mouse IgG1 antibody as described in example 1, but at different pH values. More specifically, the non-covalent binding of the anti-CD63 antibody and of the mouse IgG1 antibody to the nanoparticles were performed as described in example 1, but for a series performed at different pH values in 1 mL buffer with a pH of 2.7, of 5.5, of 6.1, of 7.5, of 9.5 and of 11.0. The different buffers were composed as follows: buffer pH 2.7: 125 mM glycine-HCl; buffer pH 5.5: 50 mM MES; buffer pH 6.1: 50 mM MES; buffer pH 7.5: 50 mM HEPES; buffer pH 9.5: 50 mM $NaHCO_3$; buffer pH 11.0: 50 mM $NaHCO_3$ and 100 mM NaOH. All buffers were adjusted with 1 N HCl or 1 N NaOH, respectively.

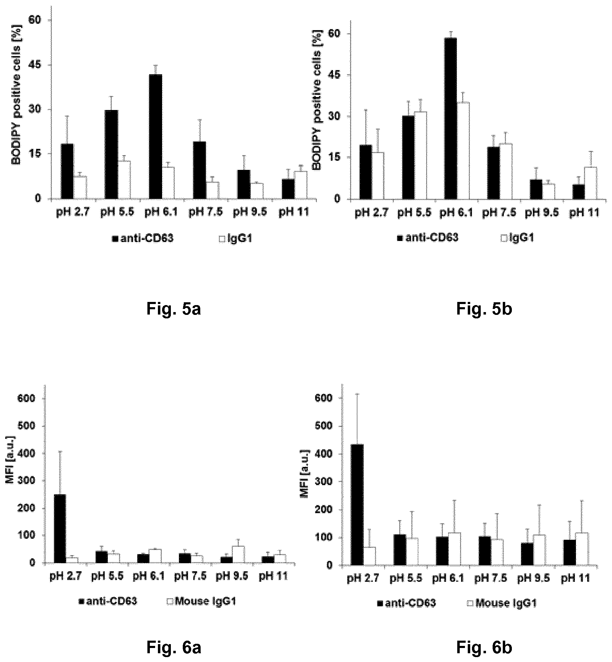

The cellular uptake or cell adhesion of each of the obtained samples in moDCs were determined. More specifically, 100,000 moDCs were treated with 7.5 $\mu$g/mL modified nanoparticles for 2 h under standard conditions. Human serum (HS) dependence was determined by using different HS concentrations, namely 0% and 10% HS during the incubation. The binding of particles was analyzed by flow cytometry at which dead cells were excluded by PI staining. The results are shown in figures 5a (for 0% HS) and 5b (for 10% HS), in which the values are shown as the amount of nanoparticle (BODIPY) positive cells in % and given as mean $\pm$ s.d. of three independent experiments.

As it derives from figures 5a and 5b, the best cellular uptake or cell adhesion is achieved in 0% HS as well as in 10% HS for the modified, carboxy group modified nanoparticles, in which the targeting moiety anti-CD63 antibody has been non-covalently bound to the surface of the nanoparticles at a pH value of 6.1. Notably, for respective nanoparticles, in which the targeting moiety anti-CD63 antibody has been non-covalently bound to the surface of the nanoparticles at a physiological pH value of 7.5, the cellular uptake or cell adhesion was drastically worse than in the case of 6.1. The same results are obtained for respective nanoparticles, in which mouse IgG1 antibody has been non-covalently bound to the surface of the nanoparticles the targeting moiety.

**Example 4 - Nanoparticles modified with amino groups and with different antibodies non-covalently bound as targeting moiety at different pH values**

(*Synthesis of amino functionalized polystyrene nanoparticles*)

Amino-functionalized polystyrene nanoparticles were synthesized via free-radical miniemulsion copolymerization according to a method as described Musyanovych et al., "Effect of hydrophilic comonomer and surfactant type on the colloidal stability and size distribution of carboxyl- and amino-functionalized polystyrene particles prepared by miniemulsion polymerization" in Langmuir 2007, volume 23, pages 5367 to 5376. 2-Aminoethyl methacrylate hydrochloride (AEMH) was used as comonomer. The nanoparticles were stabilized with the non-ionic surfactant Lutensol AT50. Briefly, 6 g freshly distilled styrene, 250 mg of hexadecane, 100 mg of initiator V59 and 6 mg BODIPY were mixed with 120 mg AEHM and 600 mg Lutensol AT50. The mixture was ultrasonicated for 2 min (450 W, 90% intensity) at 0 °C and further polymerization (at 72 °C) was carried out overnight.

The sample was subjected to a single centrifugation step (Sigma 3k30, 45 g, 7 min) and the supernatant was collected and characterized as follows: hydrodynamic diameter (Dh) = 150 $\pm$ 15 nm.

(*Non-covalently binding different targeting moieties to the nanoparticles at different pH values*)

Different antibodies have been non-covalently bound to the aforementioned nanoparticles using different buffer conditions. 200 $\mu$g of the aforementioned nanoparticles with amino groups were incubated with 100 $\mu$g anti-CD63, mouse IgG1 or mouse IgG for 4 h at room temperature in 1 mL buffer with a pH of 2.7, of 5.5, of 6.1, of 7.5, of 9.5 and of 11.0. The different buffers were composed as follows: buffer pH 2.7: 125 mM glycine-HCl; buffer pH 5.5: 50 mM MES; buffer pH 6.1: 50 mM MES; buffer pH 7.5: 50 mM HEPES; buffer pH 9.5: 50 mM $NaHCO_3$; buffer pH 11.0: 50 mM $NaHCO_3$ and 100 mM NaOH. All buffers were adjusted with 1 N HCl or 1 N NaOH, respectively.

In addition, the solutions were adjusted to 1 mL and incubated under the same conditions. The nanoparticles were centrifuged for 30 min at 20,000 g and 4 °C to remove free antibodies and resuspended with 1 mL ultrapure water. Both washing steps were repeated three times. After the last step particles were resuspended in 200 $\mu$L ultrapure water. Concentrations of all particles were determined by fluorescence intensity in the Infinite® M1000 plate reader (Tecan Group Ltd.).

(*Cellular uptake or cell adhesion in cell culture medium*)

The cellular uptake or cell adhesion of each of the samples obtained with anti CD63 antibody and mouse IgG1 in moDCs were determined. More specifically, 100,000 moDCs were treated with 9.0 μg/mL modified nanoparticles for 2 h under standard conditions. Human serum (HS) dependence was determined by using different HS concentrations, namely 0% and 10% HS during the incubation. The median fluorescence intensity (MFI) was determined by flow cytometry and values are expressed as mean ± s.d. of three independent experiments. The results are shown in figures 6a (for 0% HS) and 6b (for 10% HS), in which the values are shown as the amount of MFI.

As it derives from figures 6a and 6b, the best cellular uptake or cell adhesion is achieved in 0% HS as well as in 10% HS for the modified, amino group modified nanoparticles, in which the targeting moiety anti CD63 antibody or mouse IgG1 has been non-covalently bound to the surface of the nanoparticles at a pH value of 2.7. Notably, for respective nanoparticles, in which the targeting moiety has been non-covalently bound to the surface of the nanoparticles at a physiological pH value of 7.5, the cellular uptake or cell adhesion was drastically worse than in the case of 2.7.

**Example 5 - Nanoparticles of hydroxyethyl starch modified with different antibodies non-covalently bound as targeting moiety**

(*Synthesis of amino functionalized hydroxyethyl starch nanoparticles*)

Hydroxyethyl starch (HES) nanoparticles were synthesized as described by Baier et al., "Suppressing unspecific cellular uptake for targeted delivery using hydroxyethyl starch nanocapsules" in Biomacromolecules 2012, volume 13, pages 2704 to 2715. More specifically, HES nanoparticles were obtained using polyaddition reaction at the droplet interface via miniemulsion. HES in solution (10 wt%, 1.4 g) was mixed with 20 mg NaCl, 100 μL Cy5Oligo solution and 150 μL CellTracker. 7.5 g cyclohexane containing 100 mg of P(E/B-b-EO) was added and the mixture was stirred for 1h. Sonication was performed under ice-cooling with a Branson W450-D sonifier. Further, TDI (100 mg) and P(E/B-b-EO) (30 mg) were dissolved in 5 g cyclohexane, added dropwise and the emulsion was stirred for 24 h at RT. For purification, the nanoparticles were centrifuged and redispersion in cyclohexane (2 times). 0.1 wt% SDS was used to transfer the nanoparticles into aqueous medium. Excess SDS was removed via dialysis (24 h), centrifugation (1 time) and redispersion in water.

The sample was subjected to a single centrifugation step (Sigma 3k30, 45 g, 7 min) and the supernatant was collected and characterized as follows: hydraulic diameter (Dh) = 460 ± 50 nm.

(*Non-covalently binding a targeting moiety to the nanoparticles*)

Antibodies have been non-covalently bound to the nanoparticles by using different buffer conditions with different antibodies and at different pH values as described in example 4 except that the HES nanoparticles have been used.

(*Binding properties of the targeting moiety molecules and cellular uptake or cell adhesion in cell culture medium*)

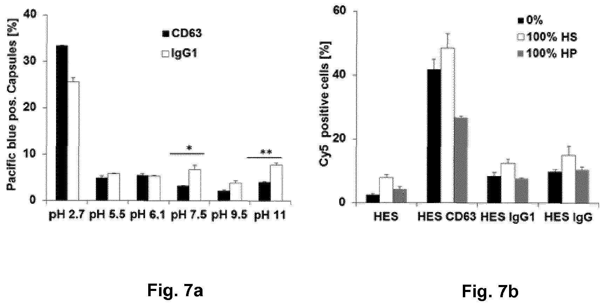

The amount of bound antibody on nanoparticles of hydroxyethyl starch obtained above at different pH values using as targeting moiety either anti-CD63 or mouse IgG1 have been determined by flow cytometry and the values are given as mean ± s.d. of three independent experiments. The results are shown in figure 7a. As it derives form figure 7a, the best binding was obtained for anti-CD63 as well as for mouse IgG1 at pH 2.7

In addition, the cellular uptake or cell adhesion of the nanoparticles of hydroxyethyl starch obtained above at different pH values using as targeting moiety either anti-CD63 or mouse IgG1 has been determined by treating 5 μg/mL of the nanoparticles with 0% plasma, with 100% human serum (HS) or with 100% human plasma (HP) and by further incubating them with moDCs for 2 h at 4 °C. The amount of nanoparticle (Cy5) positive cells was determined by flow cytometry and the values are expressed as mean ± s.d. of two independent experiments. The results are shown in figure 7b. As it derives from figure 7b, the best cellular uptake or cell adhesion is achieved in 0% HS as well as in 100% HS as well as in 100% HP for the modified nanoparticles, in which the targeting moiety anti CD63 antibody or mouse IgG1 was non-covalently bound to the surface of the nanoparticles at a pH value of 2.7. Notably, for respective nanoparticles, in which the targeting moiety was non-covalently bound to the surface of the nanoparticles at a physiological pH value of 7.5, the cellular uptake or cell adhesion was drastically worse than in the case of 2.7.

**Claims**

**1.** A modified nanoparticle for use in a therapeutic method, wherein the therapeutic method comprises the administration of the modified nanoparticle to an organism, and wherein the modified nanoparticle is obtainable by a process comprising the steps of i) providing a nanoparticle and ii) contacting the nanoparticle with one or more targeting moieties at a pH value of less than 7.0 so as to non-covalently bind the one or more targeting moieties onto the surface of the nanoparticle, wherein the nanoparticle provided in step i) is made of a material having at least one protonable or deprotonable group on the surface thereof and/or the one or more targeting moieties contacted with the nanoparticle in step ii) has at least one protonable or deprotonable group.

2.  The modified nanoparticle in accordance with claim 1, wherein the nanoparticle provided in step i) has at least one deprotonable group on the surface thereof, wherein the deprotonable group is selected from the group consisting of carboxy groups, hydroxyl groups, thiol groups, sulfenic acid groups, sulfinic acid groups, sulfonic acid groups, thiocarboxylic groups, peroxy carboxylic groups, oxime groups and arbitrary combinations of two or more of the aforementioned groups.

3.  The modified nanoparticle in accordance with claim 2, wherein the at least one deprotonable group is selected from the group consisting of carboxy groups and/or hydroxyl groups.

4.  The modified nanoparticle in accordance with claim 3, wherein the nanoparticle is made of polystyrene which is surface modified with carboxy groups, of proteins like albumin or of hydroxyethyl starch.

5.  The modified nanoparticle in accordance with any of the preceding claims, wherein the nanoparticle is contacted with the one or more targeting moieties in step ii) at a pH value of at most 6.5 and preferably at a pH value of 1.0 to 6.5.

6.  The modified nanoparticle in accordance with claim 5, wherein the nanoparticle is contacted with the one or more targeting moieties in step ii) at a pH value of 1.0 to 4.2, preferably at a pH value of 1.7 to 3.7, more preferably at a pH value of 2.1 to 3.3, yet more preferably at a pH value of 2.4 to 3.0, still more preferably at a pH value of 2.6 to 2.8 and most preferably at a pH value of about 2.7.

7.  The modified nanoparticle in accordance with claim 5, wherein the nanoparticle is contacted with the one or more targeting moieties in step ii) at a pH value of 5.0 to 6.5, preferably at a pH value of 5.5 to 6.5, more preferably at a pH value of 5.8 to 6.4, yet more preferably at a pH value of 5.9 to 6.3, still more preferably at a pH value of 6.0 to 6.2 and most preferably at a pH value of about 6.1.

8.  The modified nanoparticle in accordance with any of the preceding claims, wherein the nanoparticle provided in step i) has at least one protonable group on the surface thereof, wherein the protonable group is selected from the group consisting of amino groups, hydrazine groups, hydrazide groups, carboxy hydrazide groups, sulfonic acid amide groups, amide groups and arbitrary combinations of two or more of the aforementioned groups.

9.  The modified nanoparticle in accordance with claim 8, wherein nanoparticle is made of polystyrene, which is surface modified with amino groups and/or amide groups.

10. The modified nanoparticle in accordance with claim 8 or 9, wherein the nanoparticle is contacted with the one or more targeting moieties in step ii) at a pH value of 1.0 to 4.2, preferably at a pH value of 1.7 to 3.7, more preferably at a pH value of 2.1 to 3.3, yet more preferably at a pH value of 2.4 to 3.0, still more preferably at a pH value of 2.6 to 2.8 and most preferably at a pH value of about 2.7.

11. The modified nanoparticle in accordance with any of the preceding claims, wherein the organism is a vertebrate, a mammalian or a human being.

12. The modified nanoparticle in accordance with any of the preceding claims, wherein the therapeutic method comprises that the modified nanoparticle is administered to a biological fluid of the organism, wherein the modified nanoparticle is administered to the organism intraarterially, intravenously, intramuscularly, intrathecally, dermally, inhaled or orally.

13. Non-medical use of a modified nanoparticle, wherein the modified nanoparticle is contacted in vitro with a fluid, preferably with a biological fluid, more preferably with a protein containing biological fluid, a lipid containing fluid or a sugar containing biological fluid, and wherein the modified nanoparticle is obtainable by a process comprising the steps of i) providing a nanoparticle of a material having at least one protonable or deprotonable group on the surface thereof and ii) contacting the nanoparticle with one or more targeting moieties at a pH value of less than 7.0 so as to non-covalently bind the one or more targeting moieties onto the surface of the nanoparticle, wherein the nanoparticle provided in step i) is made of a material having at least one protonable or deprotonable group on the surface thereof and/or the one or more targeting moieties contacted with the nanoparticle in step ii) has at least one protonable or deprotonable group .

14. A method for preparing a modified nanoparticle, which comprises the steps of i) providing a nanoparticle and ii) contacting the nanoparticle with one or more targeting moieties at a pH value of less than 7.0 so as to non-covalently bind the one or more targeting moieties onto the surface of the nanoparticle, wherein the nanoparticle provided in step i) is made of a material having at least one protonable or deprotonable group on the surface thereof and/or the one or more targeting moieties contacted with the nanoparticle in step ii) has at least one protonable or deprotonable group.

15. A modified nanoparticle obtainable by a method comprising the steps of i) providing a nanoparticle and ii) contacting the nanoparticle with one or more targeting moieties at a pH value of less than 7.0 so as to non-covalently bind the one or more targeting moieties onto the surface of the nanoparticle, wherein the nanoparticle provided in step i) is made of a material having at least one protonable or deprotonable group on the surface thereof and/or the one or more targeting moieties contacted with the nanoparticle in step ii) has at least one protonable or deprotonable group.

Fig. 1a

Fig. 1b

Fig. 2

Fig. 3

Fig. 4

Fig. 5a

Fig. 5b

Fig. 6a

Fig. 6b

**Fig. 7a**

**Fig. 7b**

**EP 3 566 720 A1**

Europäisches
Patentamt

European
Patent Office

Office européen
des brevets

# EUROPEAN SEARCH REPORT

Application Number

EP 18 17 1351

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/057554 A1 (MAYO FOUNDATION [US]) 14 April 2016 (2016-04-14) * paragraphs [0040], [0056], [0075], [0112], [0138], [0152], [0176], [0200], [0221], [0229], [0230]; figures 2B,5C; examples * | 1-15 | INV. A61K47/69 A61K9/51 A61K49/18 B82Y5/00 |
| X | MEJÍAS R. ET AL.: "Cytokine adsorption/release on uniform magnetic nanoparticles for localized drug delivery", JOURNAL OF CONTROLLED RELEASE, vol. 130, 2008, pages 168-174, XP002785917, * figures 4,5C * | 1-15 | |
| Y | KOSSATZ S. ET AL.: "Efficient treatment of breast cancer xenografts with multifunctionalized iron oxide nanoparticles combining magnetic hyperthermia and anti-cancer drug delivery", BREAST CANCER RESEARCH, vol. 17, no. 66, 2015, pages 1-17, XP002785918, * figures 2b,2c * | 1-15 | TECHNICAL FIELDS SEARCHED (IPC) A61K B82Y |
| Y,D | BANNWARTH M.B. ET AL.: "Colloidal polymers with controlled sequence and branching constructed from magnetic field assembled nanoparticles", ACS NANO, vol. 9, no. 3, 2015, pages 2720-2728, XP002785919, * the whole document * | 1-15 | |

-/--

The present search report has been drawn up for all claims

| Place of search | Date of completion of the search | Examiner |
|---|---|---|
| Munich | 22 October 2018 | Giménez Miralles, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 18 17 1351

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | MUSYANOVYCH A. ET AL.: "Effect of hydrophilic comonomer and surfactant type on the colloidal stability and size distribution of carboxyl- and amino-functionalized polystyrene particles prepared by miniemulsion polymerization", LANGMUIR, vol. 23, 2007, pages 5367-5376, XP002785920, * the whole document * | 1-15 | |
| Y,D | BAIER G. ET AL.: "Suppressing unspecific cellular uptake for targeted delivery using hydroxyethyl starch nanocapsules", BIOMACROMOLECULES, vol. 13, 2012, pages 2704-2715, XP002785921, * the whole document * | 1-15 | |

TECHNICAL FIELDS
SEARCHED      (IPC)

The present search report has been drawn up for all claims

| Place of search | Date of completion of the search | Examiner |
|---|---|---|
| Munich | 22 October 2018 | Giménez Miralles, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 17 1351

This annex lists the patent family members relating to the patent documents cited in the above-mentioned European search report.
The members are as contained in the European Patent Office EDP file on
The European Patent Office is in no way liable for these particulars which are merely given for the purpose of information.

22-10-2018

| Patent document cited in search report | Publication date | Patent family member(s) | Publication date |
|---|---|---|---|
| WO 2016057554 A1 | 14-04-2016 | AU 2015328247 A1 | 30-03-2017 |
| | | AU 2018201151 A1 | 08-03-2018 |
| | | CA 2954202 A1 | 14-04-2016 |
| | | CN 107073132 A | 18-08-2017 |
| | | CR 20170031 A | 22-05-2017 |
| | | EP 3204413 A1 | 16-08-2017 |
| | | JP 2017527531 A | 21-09-2017 |
| | | KR 20170035358 A | 30-03-2017 |
| | | PE 02422017 A1 | 30-03-2017 |
| | | PH 12017500022 A1 | 15-05-2017 |
| | | SV 2017005355 A | 23-10-2017 |
| | | WO 2016057554 A1 | 14-04-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BANNWARTH et al.** Colloidal Polymers with Controlled Sequence and Branching Constructed from Magnetic Field Assembled Nanoparticles. *ACS Nano,* 2015, vol. 9, 2720-2728
- **MUSYANOVYCH et al.** Effect of hydrophilic comonomer and surfactant type on the colloidal stability and size distribution of carboxyl- and amino-functionalized polystyrene particles prepared by miniemulsion polymerization. *Langmuir,* 2007, vol. 23, 5367-5376
- **BAIER et al.** Suppressing unspecific cellular uptake for targeted delivery using hydroxyethyl starch nanocapsules. *Biomacromolecules,* 2012, vol. 13, 2704-2715